# EUROPÄISCHE PATENTSCHRIFT

(11) **EP 0 708 095 B1**
(45) Veröffentlichungstag und Bekanntmachung des Hinweises auf die Patenterteilung: **19.05.1999**
(21) Anmeldenummer: 95114212.4
(22) Anmeldetag: 11.09.1995
(51) Int. Cl.: C07D 249/12, C07C 281/06, A01N 47/38

(54) **Verfahren zur Herstellung von alkoxytriazolinonen**
Process for the preparation of alkoxytriazoles
Procédé de préparation d'alcoxytriazoles

(30) Priorität: 23.09.1994 DE 4433967
(43) Veröffentlichungstag der Anmeldung: 24.04.1996
(73) Patentinhaber: BAYER AG, 51368 Leverkusen (DE)
(72) Erfinder: Wroblowsky, Heinz-Jürgen, Dr., D-40764 Langenfeld (DE); König, Klaus, Dr., D-51519 Odenthal (DE)

(56) Entgegenhaltungen:
- EP-A- 0 477 646
- EP-A- 0 507 171
- WO-A-94/08979
- DE-A- 1 940 367
- ARCH. PHARM. (WEINHEIM, GER.) (ARPMAS);74; VOL.307 (11); PP.889-91, TECH. UNIV. BRAUNSCHWEIG;INST. PHARM. CHEM.; BRAUNSCHWEIG; GER., ZINNER G ET AL 'Reaction of cyanates with carbonate hydrazides'
- J. CHEM. SOC., PERKIN TRANS. 1 (JCPRB4);73; (22); PP.2644-6, IMP. CHEM. IND. LTD.;JEALOTT'S HILL RES. STN.; BRACKNELL; ENGL., ATKINS P R ET AL 'Heterocyclic syntheses with isothiocyanatoformic esters and their derivatives'
- REV. FAC. SCI. ISTANBUL, Bd. 13a, 1948 ISTANBUL, Seiten 127-144, F.ARNDT ET AL 'Tautomerie und Methylderivate des Urazols'

## Beschreibung

Die Erfindung betrifft ein neues, auch technisch durchführbares Verfahren zur Herstellung von weitgehend bekannten Alkoxytriazolinonen, welche als Zwischenprodukte zur Herstellung von agrochemischen Wirkstoffen verwendet werden können.

Alkoxytriazolinone und mehrere Methoden zu ihrer Herstellung sind bereits bekannt (vgl. J. Indian Chem. Soc. 6 (1929), 565-575; J. Chem. Soc. Perkin I 1973, 2644-2646; Arch. Pharm. 307 (1974), 889-891; EP-A 477646; EP-A 507171). Nach diesen bekannten Synthesemethoden werden Alkoxytriazolinone jedoch nur in sehr unbefriedigenden Ausbeuten erhalten.

Ferner ist bekannt, daß durch Methylierung von Urazol oder 4-Methylurazol mit Diazomethan (CH₂N₂) 5-Methoxy-4-methyl-2,4-dihydro-3H-1,2,4-triazol-3-on gebildet wird (vgl. F. Arndt et al., Rev. Fac. Sci. Istanbul 13A, S. 127-144 (1948)); diese Methode liefert zwar das Triazolinon in hoher Ausbeute, kann aber technisch nicht durchgeführt werden.

Es wurde nun gefunden, daß man Alkoxytriazolinone der allgemeinen Formel (I) 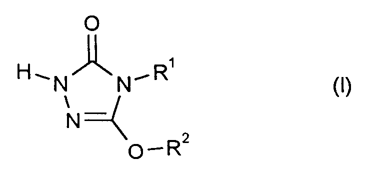 in welcher
- R¹: für jeweils gegebenenfalls durch Cyano, Halogen oder C₁-C₄-Alkoxy substituiertes Alkyl, Alkenyl oder Alkinyl mit jeweils bis zu 6 Kohlenstoffatomen, für jeweils gegebenenfalls durch Halogen oder C₁-C₄-Alkyl substituiertes Cycloalkyl oder Cycloalkylalkyl mit jeweils 3 bis 6 Kohlenstoffatomen im Cycloalkylteil und gegebenenfalls 1 bis 4 Kohlenstoffatomen im Alkylteil, oder für jeweils gegebenenfalls durch Carboxy, Cyano, Nitro, Halogen, C₁-C₄-Alkyl, C₁-C₄-Halogenalkyl, C₁-C₄-Alkoxy, C₁-C₄-Halogenalkoxy oder C₁-C₄-Alkoxy-carbonyl substituiertes Aryl oder Arylalkyl mit jeweils 6 oder 10 Kohlenstoffatomen im Arylteil und gegebenenfalls 1 bis 4 Kohlenstoffatomen im Alkylteil steht und
- R²: für jeweils gegebenenfalls durch Halogen oder C₁-C₄-Alkoxy substituiertes Alkyl, Alkenyl oder Alkinyl mit jeweils bis zu 6 Kohlenstoffatomen, für jeweils gegebenenfalls durch Halogen oder C₁-C₄-Alkyl substituiertes Cycloalkyl oder Cycloalkylalkyl mit jeweils 3 bis 6 Kohlenstoffatomen im Cycloalkylteil und gegebenenfalls 1 bis 4 Kohlenstoffatomen im Alkylteil, oder für jeweils gegebenenfalls durch Carboxy, Cyano, Nitro, Halogen, C₁-C₄-Alkyl, C₁-C₄-Halogenalkyl, C₁-C₄-Alkoxy, C₁-C₄-Halogenalkoxy oder C₁-C₄-Alkoxy-carbonyl substituiertes Aryl oder Arylalkyl mit jeweils 6 oder 10 Kohlenstoffatomen im Arylteil und gegebenenfalls 1 bis 4 Kohlenstoffatomen im Alkylteil steht,
in sehr guten Ausbeuten und in hoher Reinheit erhält, wenn man Iminokohlensäurediester der allgemeinen Formel (II) 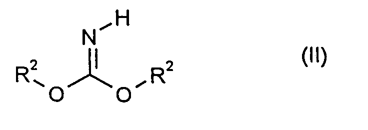 in welcher
- R²: die oben angegebene Bedeutung hat,
mit Carbazinsäureestern der allgemeinen Formel (III) 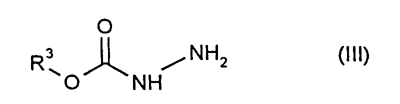 in welcher
- R³: für gegebenenfalls durch C₁-C₄-Alkoxy substituiertes C₁-C₄-Alkyl, für Phenyl oder Benzyl steht,
gegebenenfalls in Gegenwart eines Reaktionshilfsmittels und gegebenenfalls in Gegenwart eines Verdünnungsmittels bei Temperaturen zwischen -20°C und +120°C umsetzt ("erste Umsetzungsstufe") und die hierbei gebildeten Semicarbazid-Derivate der allgemeinen Formel (IV) 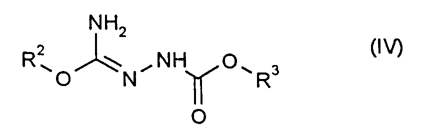 in welcher
- R² und R³: die oben angegebene Bedeutung haben,
- und/oder die entsprechenden hierzu tautomeren Verbindungen -,
gegebenenfalls nach Zwischenisolierung, gegebenenfalls in Gegenwart einer Base und gegebenenfalls in Gegenwart eines Verdünnungsmittels bei Temperaturen zwischen 20°C und 150°C cyclisierend kondensiert ("zweite Umsetzungsstufe") und schließlich die so erhaltenen Alkoxytriazolinone der allgemeinen Formel (V) 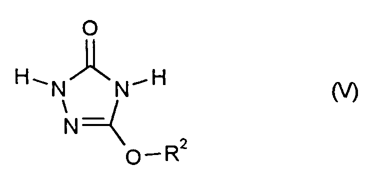 in welcher
- R²: die oben angegebene Bedeutung hat,
- und/oder die entsprechenden hierzu tautomeren Verbindungen -
mit einem Alkylierungsmittel der allgemeinen Formel (VI)

R¹-X (VI)

worin
- X: für Halogen oder die Gruppierungen -O-SO₂-O-R¹ oder -O-CO-O-R¹ steht und
- R¹: die oben angegebene Bedeutung hat,
gegebenenfalls in Gegenwart einer Base und gegebenenfalls in Gegenwart eines Verdünnungsmittels bei Temperaturen zwischen 0°C und 150°C umsetzt ("dritte Umsetzungsstufe").

Überraschenderweise können die Alkoxytriazolinone der allgemeinen Formel (I) nach dem erfindungsgemäßen Verfahren in erheblich höheren Ausbeuten als nach den meisten bekannten Synthesemethoden erhalten werden. Gegenüber der "Diazomethan-Methode" (F. Arndt et al., l.c.) hat das erfindungsgemäße Verfahren den entscheidenden Vorteil, daß es auch technisch durchführbar ist.

Es ist vor allem als überraschend anzusehen, daß die Alkylierung der Verbindungen der Formel (V) in der dritten Stufe in hoher Selektivität am N-Atom in Position 4 und nicht an einem der anderen N-Atome bzw. am Carbonyl-Sauerstoff erfolgt.

Die Ausdrücke "Alkylierung" und "Alkylierungsmittel" (VI) werden in diesem Zusammenhang als Oberbegriffe verwendet und schließen damit ausdrücklich alle Möglichkeiten ein, welche sich aus der obigen Definition von R¹ ergeben (d.h. neben R¹ = Alkyl, Cycloalkylalkyl und Arylalkyl auch R¹ = Alkenyl, Alkinyl, Cycloalkyl und Aryl).

Da die benötigten Ausgangsstoffe der Formeln (II) und (III) relativ einfach herzustellende, kostengünstige Chemikalien sind und die erfindungsgemäßen Umsetzungen glatt und mit hohen Ausbeuten ablaufen, stellt das erfindungsgemäße Verfahren eine wertvolle Bereicherung des Standes der Technik dar.

In einer möglichen Ausführungsform des erfindungsgemäßen Verfahrens lassen sich alle Stufen "im Eintopf", d.h. ohne Zwischenisolierung der Intermediate durchführen.

Die Erfindung betrifft vorzugsweise die Herstellung von Verbindungen der Formel (I), in welcher
- R¹: für jeweils gegebenenfalls durch Cyano, Fluor, Chlor und/oder Brom, Methoxy oder Ethoxy substituiertes Methyl, Ethyl, n- oder i-Propyl, n-, i-, s- oder t-Butyl, für jeweils gegebenenfalls durch Cyano, Fluor, Chlor und/oder Brom substituiertes Propenyl, Butenyl, Propinyl oder Butinyl, für jeweils gegebenenfalls durch Fluor, Chlor, Brom, Methyl oder Ethyl substituiertes Cyclopropyl, Cyclobutyl oder Cyclopropylmethyl, oder für jeweils gegebenenfalls durch Cyano, Fluor, Chlor, Brom, Methyl, Ethyl, Trifluormethyl, Methoxy, Ethoxy, Difluormethoxy, Trifluormethoxy, Methoxycarbonyl oder Ethoxycarbonyl substituiertes Phenyl oder Benzyl steht und
- R²: für jeweils gegebenenfalls durch Fluor, Chlor und/oder Brom, Methoxy oder Ethoxy substituiertes Methyl, Ethyl, n- oder i-Propyl, n-, i-, s- oder t-Butyl, für jeweils gegebenenfalls durch Cyano, Fluor, Chlor und/oder Brom substituiertes Propenyl, Butenyl, Propinyl oder Butinyl, für jeweils gegebenenfalls durch Fluor, Chlor, Methyl oder Ethyl substituiertes Cyclopropyl oder Cyclopropylmethyl, oder für jeweils gegebenenfalls durch Cyano, Fluor, Chlor, Brom, Methyl, Ethyl, Trifluormethyl, Methoxy, Ethoxy, Difluormethoxy, Trifluormethoxy, Methoxycarbonyl oder Ethoxycarbonyl substituiertes Phenyl oder Benzyl steht.
Verwendet man beispielsweise Imino-kohlensäure-dimethylester und Carbazinsäure-ethylester sowie Methylbromid als Ausgangsstoffe, so kann der Reaktionsablauf beim erfindungsgemäßen Verfahren durch das folgende Formelschema skizziert werden:

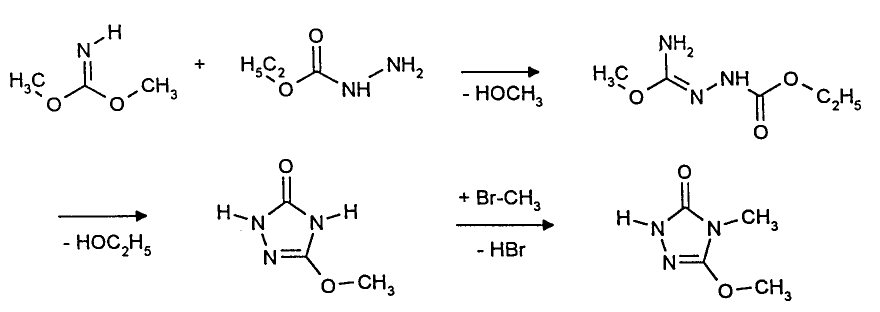

Die beim erfindungsgemäßen Verfahren zur Herstellung der Verbindungen der allgemeinen Formel (I) als Ausgangsstoffe zu verwendenden Iminokohlensäurediester sind durch die Formel (II) allgemein definiert. In der Formel (II) hat R² vorzugsweise diejenige Bedeutung, die bereits oben im Zusammenhang mit der Beschreibung der Verbindungen der Formel (I) als bevorzugt für R² angegeben wurde.

Die Ausgangsstoffe der Formel (II) sind bekannt und/oder können nach an sich bekannten Verfahren hergestellt werden (vgl. Chem. Ber. 46 (1913), 2447; J. Prakt. Chem. 315 (1973), 640-648; DE-A 1518230; DE-A 4123608).

Die beim erfindungsgemäßen Verfahren weiter als Ausgangsstoffe zu verwendenden Carbazinsäureester sind durch die Formel (III) allgemein definiert. In der Formel (III) steht R³ vorzugsweise für Methyl, Ethyl, Methoxyethyl, Ethoxyethyl oder Phenyl.

Die Ausgangsstoffe der Formel (III) sind bekannte organische Synthesechemikalien.

Die in der ersten Stufe des erfindungsgemäßen Verfahrens als Zwischenprodukte gebildeten Semicarbazid-Derivate der Formel (IV) sind neu, ausgenommen die Verbindungen, in welchen
- R²: für Phenyl und R³ für Methyl oder tert.-Butyl steht;
- R²: für 2.2.2-Trichlorethyl und R³ für Methyl, Ethyl oder tert.-Butyl steht; und
- R²: für 2.2.2-Trifluorethyl und R³ für Methyl, Ethyl oder tert.-Butyl steht.

Diese acht Semicarbazid-Derivate, hergestellt nach einem anderen Verfahren, sind vorbeschrieben (vgl. G. Zinner, Arch. Pharm. 307, S. 889-891 (1974)).

Die in der zweiten Stufe des erfindungsgemäßen Verfahrens als Zwischenprodukte gebildeten 5-Alkoxytriazolinone der Formel (V) sind ebenfalls neu, ausgenommen die Verbindungen, in welchen
- R²: für Methyl, Ethyl, Phenyl, 4-Methylphenyl, 2,4-Dimethylphenyl oder 4-tert.-Butylphenyl steht.

Diese sechs Alkoxytriazolinone, hergestellt jeweils nach anderen, unterschiedlichen Verfahren, sind vorbeschrieben (vgl. J. Chem. Soc., Perkin Trans. I, S. 2644-2646 (1973) betreffend R² = CH₃; Arch. Pharm. 307, S. 889-891 (1974) betreffend R² = C₂H₅; DE-A-19 40 367 betreffend R² = C₆H₅ und substituiertes Phenyl wie oben angegeben).

Die neuen Semicarbazid-Derivate der Formel (IV) und die neuen Alkoxytriazolinone der Formel (V) als solche sind auch Gegenstand der vorliegenden Erfindung.

Die beim erfindungsgemäßen Verfahren ferner als Ausgangsstoffe zu verwendenden Alkylierungsmittel sind durch die Formel (VI) allgemein definiert. In der Formel (VI) hat R¹ vorzugsweise diejenige Bedeutung, die bereits oben im Zusammenhang mit der Beschreibung der Verbindungen der Formel (I) als bevorzugt für R¹ angegeben wurde.

Die Ausgangsstoffe der Formel (VI) sind bekannte organische Synthesechemikalien.

Als Verdünnungsmittel zur Durchführung des erfindungsgemäßen Verfahrens kommen (in allen Umsetzungsstufen) die üblichen organischen Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methyl-pyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, n-, i-, s- oder t-Butanol, Ethylenglykolmonomethylether, Ethylenglykolmonoethylether, Diethylenglykolmonomethylether, Diethylenglykolmonoethylether, deren Gemische mit Wasser oder reines Wasser.

Alkohole, wie Methanol, Ethanol, n- oder i-Propanol werden als Verdünnungsmittel in der ersten Stufe besonders bevorzugt.

Das erfindungsgemäße Verfahren wird in der ersten Stufe vorzugsweise in Gegenwart eines geeigneten Reaktionshilfsmittels durchgeführt. Als solche kommen vorzugsweise Protonensäuren, wie z.B. Salzsäure, Schwefelsäure, Phosphorsäure, Kohlensäure, Essigsäure, Propionsäure, Pivalinsäure, Methansulfonsäure, Benzoesäure, Benzolsulfonsäure und p-Toluolsulfonsäure - gegebenenfalls auch polymere Säuren oder saure Ionenaustauscher - in Betracht.

Pivalinsäure, Essigsäure und (wässrige) Salzsäure werden als Reaktionshilfsmittel bei der ersten Stufen des erfindungsgemäßen Verfahrens besonders bevorzugt.

Das erfindungsgemäße Verfahren wird in der zweiten und dritten Stufe vorzugsweise in Gegenwart einer Base durchgeführt. Es kommen hierbei alle üblichen anorganischen oder organischen Basen in Betracht. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie basische organische Stickstoffverbindungen, wie Trimethylamin, Triethylamin, Tributylamin, N,N-Dimethyl-anilin, N,N-Dimethyl-benzylamin, Pyridin, N-Methylpiperidin, N,N-Dimethylaminopyridin, 5-Ethyl-2-methyl-pyridin, Diazabicyclooctan (DABCO), Diazabicyclononen (DBN) oder Diazabicycloundecen (DBU).

Alkalimetall-hydroxide, wie Natrium- oder Kaliumhydroxid, -alkoholate, wie Natriummethylat oder Natriumethylat, oder -carbonate, wie Natrium- oder Kaliumcarbonat werden als Basen bei der zweiten Stufe des erfindungsgemäßen Verfahrens besonders bevorzugt.

Die Reaktionstemperaturen können bei der Durchführung der ersten Stufe des erfindungsgemäßen Verfahrens in einem größeren Bereich variiert werden. Im allgemeinen arbeitet man bei Temperaturen zwischen -20°C und +120°C, vorzugsweise bei Temperaturen zwischen -10°C und 90°C, insbesondere bei Temperaturen zwischen 0°C und 60°C.

Die Reaktionstemperaturen können bei der Durchführung der zweiten Stufe des erfindungsgemäßen Verfahrens in einem größeren Bereich variiert werden. Im allgemeinen arbeitet man bei Temperaturen zwischen 20°C und 150°C, vorzugsweise bei Temperaturen zwischen 30°C und 90°C, insbesondere bei Temperaturen zwischen 40°C und 80°C.

Die Reaktionstemperaturen können bei der Durchführung der dritten Stufe des erfindungsgemäßen Verfahrens in einem größeren Bereich variiert werden. Im allgemeinen arbeitet man bei Temperaturen zwischen 0°C und 150°C, vorzugsweise bei Temperaturen zwischen 30°C und 90°C, insbesondere bei Temperaturen zwischen 40°C und 80°C.

Das erfindungsgemäße Verfahren wird in allen Stufen im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens zur Herstellung der Verbindungen der Formel (I) setzt man pro Mol Iminokohlensäurediester der Formel (II) im allgemeinen 0,5 bis 1,2 Mol, vorzugsweise 0,8 bis 1,1 Mol Carbazinsäureester der Formel (III) und 1,0 bis 3,0 Mol, vorzugsweise 1,05 bis 1,50 Mol Alkylierungsmittel der Formel (VI) ein.

In einer bevorzugten Ausführungsform des erfindunggemäßen Verfahrens werden die Ausgangsstoffe der Formel (II) und der Formel (III) sowie gegebenenfalls ein Reaktionshilfsmittel in einem geeigneten Verdünnungsmittel vermischt und bei der erforderlichen Temperatur gerührt, bis praktisch kein Ausgangsmaterial mehr vorhanden ist. Die Isolierung des Zwischenproduktes der Formel (IV) kann dann auf übliche Weise, beispielsweise durch Einengen, Digerieren des Rückstandes mit einem organischen Lösungsmittel, wie z.B. Methyl-t-butylether, und Absaugen durchgeführt werden. Das Zwischenprodukt der Formel (IV) kann aber auch ohne Zwischenisolierung mit einer Base - gegebenenfalls in einem der oben angegebenen Verdünnungsmittel gelöst - versetzt und bei der zur cyclisierenden Kondensation erforderlichen Temperatur bis zum Ende der Umsetzung gerührt werden. Vor der Durchführung der letzten Umsetzungsstufe wird das Zwischenprodukt der Formel (V) vorzugsweise nicht isoliert. Es kann jedoch - falls gewünscht - isoliert werden, beispielsweise indem man einengt, den Rückstand in gesättigter wässriger Kochsalzlösung aufnimmt, mit etwa der äqimolaren Menge einer Säure, wie z.B. Salzsäure behandelt, absaugt und das Festprodukt trocknet. Zur Alkylierung wird das so erhaltene Produkt vorzugsweise in einem der oben angegebenen Lösungsmittel aufgenommen, mit einer Base und einem Alkylierungsmittel der Formel (VI) versetzt und bis zum Ende der Umsetzung bei der erforderlichen Temperatur gerührt.

Das Zwischenprodukt der Formel (IV) kann aber auch nach Isolierung direkt im Eintopfverfahren durch alkalischen Ringschluß und, eventuell nach einem Lösungsmittelwechsel, mit einem Alkylierungsmittel (VI) zum Alkoxytriazolinon (I) umgesetzt werden.

Die gesamte Synthesesequenz kann aber auch ohne Isolierung der Zwischenprodukte durchgeführt werden.

Die Aufarbeitung zur Isolierung der Produkte der Formel (I) kann nach üblichen Methoden durchgeführt werden. Beispielsweise wird filtriert und das Filtrat eingeengt, der Rückstand in einem organischen Lösungsmittel, wie z.B. Methylenchlorid aufgenommen und über Kieselgel filtriert. Nach sorgfältigem Abdestillieren des Lösungsmittels unter vermindertem Druck wird dann das Produkt der Formel (I) als Rückstand erhalten.

Alternativ kann man das Reaktionsgemisch nach Ablauf der Alkylierungsreaktion in jeweiligen Lösungsmittel auf Rückflußtemperatur erhitzen und die Anorganica durch Heißfiltration abtrennen. Durch Abkühlen des Filtrats, das gegebenenfalls zunächst durch teilweises Abdestillieren des Lösungsmittels stärker konzentriert wird, erhält man die Produkte (I) als Niederschlag, der abgesaugt und getrocknet wird.

Die nach dem erfindungsgemäßen Verfahren herzustellenden Verbindungen der Formel (I) können als Zwischenprodukte zur Herstellung von herbizid wirksamen Verbindungen verwendet werden (vgl. EP-A 477646 und EP-A 507171).

### Herstellungsbeispiele:

### Beispiel 1

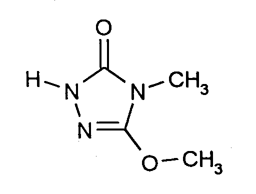

### Stufen 1 und 2:

53,6 g (0,5 Mol) Carbazinsäure-ethylester werden in 100 ml Methanol gelöst und nach Zugabe von 1,0 g (0,01 Mol) Pivalinsäure werden bei 0°C 213 g einer 23%igen Lösung von Iminokohlensäure-dimethylester (0,55 Mol) in Methanol langsam eindosiert. Das Gemisch wird 2 Stunden bei 0°C und weitere 6 Stunden bei 20°C gerührt. Dann werden 90 g einer 30%igen Lösung von Natriummethanolat (0,5 Mol) in Methanol dazu gegeben und die Reaktionsmischung wird 15 Stunden bei 55°C gerührt. Anschließend wird eingeengt, der Rückstand in 150 ml gesättigter wässriger Kochsalzlösung aufgenommen und bei 0°C mit 0,5 Mol konz. Salzsäure tropfenweise versetzt. Nach 10 Minuten bei 0°C wird abgesaugt und der erhaltene Feststoff getrocknet.

Man erhält (nach Gehaltsbestimmung) 38,9 g (68% der Theorie) 5-Methoxy-2,4-dihydro-3H-1,2,4-triazol-3-on vom Schmelzpunkt 220°C.

### Stufe 3

10,0 g (87 mMol) 5-Methoxy-2,4-dihydro-3H-1,2,4-triazol-3-on werden in 120 ml Acetonitril gelöst und nach Zugabe von 12,6 g (91 mMol) Kaliumcarbonat bei 55°C mit 11,5 g (91 mMol) Dimethylsulfat tropfenweise versetzt. Das Reaktionsgemisch wird 2 Stunden bei 55°C gerührt und dann filtriert. Das Filtrat wird eingeengt, der Rückstand in Methylenchlorid gelöst und über Kieselgel filtriert. Vom Filtrat wird das Lösungsmittel unter vermindertem Druck sorgfältig abdestilliert.

Der Rückstand wird aus Wasser umkristallisiert.

Man erhält 8,4 g (73% der Theorie - bezogen auf das zur dritten Stufe eingesetzte Ausgangsmaterial) 5-Methoxy-4-methyl-2,4-dihydro-3H-1,2,4-triazol-3-on vom Schmelzpunkt 148°C.

### Beispiel 2 (nur dritte Stufe)

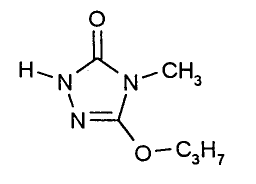

10,0 g (70 mMol) 5-Propoxy-2,4-dihydro-3H-1,2,4-tiazol-3-on (vgl. Beispiel V-1) werden in 120 ml Acetonitril gelöst und nach Zugabe von 10,1 g (73 mMol) Kaliumcarbonat bei 55°C mit 9,2 g (73 mMol) Dimethylsulfat tropfenweise versetzt. Das Reaktionsgemisch wird 6 Stunden bei 55°C gerührt und dann filtriert. Das Filtrat wird eingeengt, der Rückstand in Methylenchlorid gelöst und über Kieselgel filtriert. Vom Filtrat wird das Lösungsmittel unter vermindertem Druck sorgfältig abdestilliert.

Man erhält 10,5 g (90% der Theorie) 4-Methyl-5-propoxy-2,4-dihydro-3H-1,2,4-triazol-3-on als amorphes Produkt.

### Beispiel 3

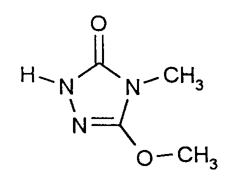

### ("Eintopf-Methode")

42,8 g (0,4 Mol) Carbazinsäure-ethylester werden in 40 ml Methanol vorgelegt und nach Zugabe von 128,4 ml einer methanolischen Lösung von 0,44 Mol Iminokohlensäure-dimethylester auf 0°C abgekühlt. Nach Zugabe von 0,8 ml konz. Salzsäure (0,008 Mol HCl) wird die Mischung 2 Stunden bei 0°C und dann noch 24 Stunden bei 20°C gerührt. Anschließend werden 89,5 g einer methanolischen Lösung von Natriummethylat (0,42 Mol NaOCH₃) eindosiert und das Gemisch wird 12 Stunden bei 55°C bis 60°C gerührt. Dann wird auf 20°C abgekühlt und 37,9 g (0,4 Mol) Dimethylsulfat werden tropfenweise eindosiert. Das Reaktionsgemisch wird 2 Stunden bei 40°C gerührt, mit weiteren 3,8 g (0,04 Mol) Dimethylsulfat versetzt und weitere 2 Stunden bei 40°C gerührt. Dann wird im Wasserstrahlvakuum eingeengt, der Rückstand in 120 ml Wasser aufgenommen und im Eisbad mit konz. Salzsäure angesäuert. Das kristallin angefallene Produkt wird durch Absaugen isoliert.

Man erhält 33,7 g 5-Methoxy-4-methyl-2,4-dihydro-3H-1,2,4-triazol-3-on (Gehalt: 90%, Ausbeute: 59% der Theorie über alle Stufen).

Die nachfolgenden Beispiele 4 bis 6 zeigen die Durchführung der zweiten und dritten Stufe im Eintopfverfahren:

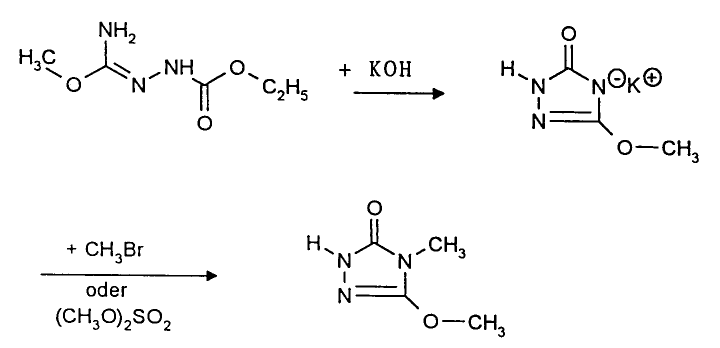

### Beispiel 4: Methylierung mit Methylbromid

50 g (0,306 Mol) N'-(α-Amino-α-methoxy-methylen)-hydrazin-N-carbonsäureethylester (Gehalt: 98,5 %) werden zu einer Lösung von 20,4 g (0,321 Mol) 88 %igem Kaliumhydroxid in 220 ml Methanol gegeben und bei 55°C über Nacht gerührt. Anschließend entfernt man das Methanol im Vakuum, nimmt den Rückstand mit 300 ml Propionsäurenitril auf, kühlt auf -10°C ab, kondensiert 32 g (0,336 Mol) Methylbromid ein und rührt 6 Stunden bei 55°C unter Eigendruck. Dann wird das Druckgefäß entspannt und das Reaktionsgemisch wird auf Rückflußtemperatur erhitzt und heiß von dem unlöslichen Kaliumbromid abfiltriert. Das Filtrat wird auf ca. 100 ml eingeengt und auf -15°C abgekühlt; das hierbei ausgefallene Produkt wird abfiltriert und im Vakuum getrocknet.

Man erhält 28,8 g (72 % der Theorie) 5-Methoxy-4-methyl-2,4-dihydro-3H-1,2,4-triazol-3-on (Gehalt nach HPLC gegen Standard: 99 %) vom Schmelzpunkt 146°C.

### Beispiel 5: Methylierung mit Methylbromid

70 g (0,428 Mol) N'-(α-Amino-α-methoxy-methylen)-hydrazin-N-carbonsäureethylester (Gehalt: 98,5 %) werden zu einer Lösung von 28,4 g (0,446 Mol) 88 %igem Kaliumhydroxid in 300 ml Methanol gegeben und über Nacht bei 55°C gerührt. Anschließend entfernt man das Lösungmittel im Vakuum, nimmt den Rückstand in 250 ml Methyl-isobutylketon auf, kühlt auf -10°C ab, kondensiert 44,4 g (0,467 Mol) Methylbromid ein und rührt 6 Stunden bei 55°C unter Eigendruck.

Zur Ausbeutebestimmung wird nach dem Entspannen des Druckgefaßes zur Trockne eingedampft und das Rohprodukt (Auswaage: 103,6 g) im Mörser zerkleinert.

Gehalt an 5-Methoxy-4-methyl-2,4-dihydro-3H-1,2,4-triazol-3-on gegen Standard: 47 Gew.-% (entspricht einer Ausbeute von 89 % der Theorie);
Gehalt an KBr: 45,2 Gew.-%.

### Beispiel 6: Methylierung mit Dimethylsulfat

50 g (0,306 Mol) N'-(α-Amino-α-methoxy-methylen)hydrazin-N-carbonsäure-ethylester (Gehalt: 98,5 %) werden zu einer Lösung von 20,4 g (0,321 Mol) 88 %igem Kaliumhydroxid in 250 ml Methanol gegeben und über Nacht bei 55°C gerührt. Anschließend wird eingeengt, der Rückstand in 270 ml Methyl-isobutylketon aufgenommen, und bei 55°C werden innerhalb von 2 Stunden 40,5 g (0,321 Mol) Dimethylsulfat zugetropft. Nach vollendeter Zugabe läßt man noch 2 Stunden bei 55°C nachrühren, engt dann auf etwa ein Drittel des ursprünglichen Volumens ein und filtriert die Feststoffe ab. Zur Abtrennung der Anorganica aus dem Filterrückstand erhitzt man die Feststoffe mit 200 ml Propionsäurenitril auf Rückfluß und filtriert heiß ab. Das Filtrat wird eingedampft und der feste Rückstand wird im Vakuum getrocknet.

Man erhält 29,2 g (69,5 % der Theorie) 5-Methoxy-4-methyl-3H-1,2,4-triazol-3-on (Gehalt: 94 %).

### Zwischenprodukte der Formel (IV):

### Beispiel (IV-1)

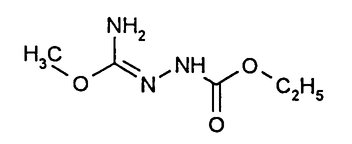

21,1 g (0,2 Mol) Carbazinsäure-ethylester werden in 20 ml Methanol vorgelegt und nach Zugabe von 61,3 ml einer methanolischen Lösung von Iminokohlensäuredimethylester mit einem Gehalt an Diester von 305 g/l (= 0,21 Mol Diester) auf 0°C abgekühlt. Dann werden 0,4 ml konz. Salzsäure (0,004 Mol HCl) dazu gegeben und das Gemisch wird 6 Stunden bei 0°C und weitere 15 Stunden bei 20°C gerührt. Nach Zugabe von weiteren 2,9 ml der methanolischen Lösung von Iminokohlensäure-dimethylester wird die Mischung weitere 6 Stunden bei 20°C gerührt. Dann wird im Wasserstrahlvakuum eingeengt, der Rückstand mit 220 ml t-Butyl-methyl-ether verrührt und das kristalline Produkt durch Absaugen isoliert.

Man erhält 30,1 g (90% der Theorie) N'-(α-Amino-α-methoxy-methylen)-hydrazin-N-carbonsäure-ethylester (Gehalt: 96,1%).

¹H-NMR (Dimethylsulfoxid-D₆): 1,165 ppm (3H, Triplett); 3,573 ppm (3H, Singulett); 3,994 ppm (2H, Quartett); 5,887 ppm (2H, Singulett); 8,475 ppm (1H, Singulett).

### Beispiel (IV-2)

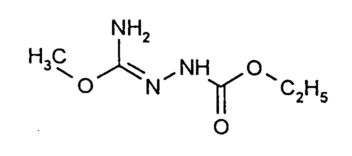

21,1 g (0,2 Mol) Carbazinsäure-ethylester werden in 20 ml Methanol vorgelegt und nach Zugabe von 61,3 ml einer methanolischen Lösung von Iminokohlensätiredinlethylester mit einem Gehalt an Diester von 305 g/l (= 0,21 Mol Diester) auf 0°C abgekühlt. Dann werden 0,24 g (0,004 Mol) Essigsäure in 2 ml Methanol zugetropft. Die Mischung wird 6 Stunden bei 0°C und weitere 15 Stunden bei 20°C gerührt. Dann wird im Wasserstrahlvakuum eingeengt, der Rückstand mit t-Butyl-methyl-ether verrührt und das kristalline Produkt durch Absaugen isoliert.

Man erhält 30,8 g (93% der Theorie) N'-(α-Amino-α-methoxy-methylen)-hydrazin-N-carbonsäure-ethylester (Gehalt: 97,5%) vom Schmelzpunkt 134°C.

### Beispiel (IV-3)

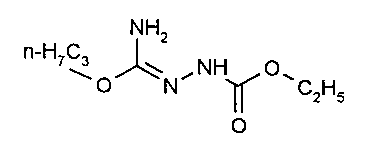

6,0 g (0,041 Mol) Iminokohlensäure-dipropylester und 4,07 g (0,038 Mol) Carbazinsäure-ethylester werden in 20 ml Methanol gelöst und eine Lösung von 0,19 g (0,0019 Mol) Pivalinsäure in 2 ml Methanol wird bei 20°C tropfenweise dazu gegeben. Die Mischung wird noch 15 Stunden bei 20°C gerührt. Dann wird im Wasserstrahlvakuum eingeengt, der Rückstand mit 30 ml t-Butyl-methyl-ether verrührt und das kristalline Produkt durch Absaugen isoliert.

Man erhält 5,54 g (75% der Theorie) N'-(α-Amino-α-n-propoxy-methylen)-hydrazin-N-carbonsäure-ethylester (Gehalt: 96,7%) vom Schmelzpunkt 100°C.

### Beispiel (IV-4)

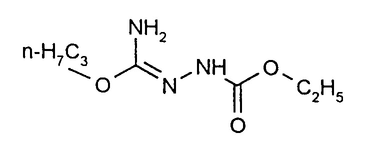

26,8 g (0,25 Mol) Carbazinsäure-ethylester (Gehalt: 97 %) und 48,4 g (0,30 Mol) Iminokohlensäure-di-n-propylester (Gehalt: 90 %) werden in 120 ml n-Propanol bei Raumtemperatur vorgelegt, und innerhalb von 1,5 Stunden wird eine Lösung von 1,53 g (0,015 Mol) Pivalinsäure in 40 ml n-Propanol zugetropft. Nach beendeter Zugabe läßt man über Nacht nachrühren und gibt dann weitere 4,8 g (0,03 Mol) Iminokohlensäure-dipropylester und 0,5 g Pivalinsäure zum Reaktionsgemisch. Nach weiteren 4 StundenRühren bei Raumtemperatur engt man ein, versetzt den Rückstand mit 250 ml Petrolether, rührt 1,5 Stunden bei Raumtemperatur und filtriert das Produkt ab.

Man erhält 41,75 g (88,1 % der Theorie) N'-(α-Amino-α-n-propoxymethylen)-hydrazin-N-carbonsäure-ethylester (Gehalt: 98,7 %).

### Zwischenprodukte der Formel (V):

### Beispiel (V-1)

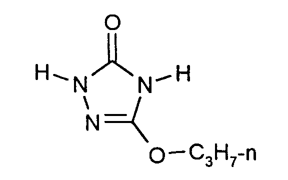

6,82 g (0,0349 Mol) N'-(α-Amino-α-n-propoxy-methylen)-hydrazin-N-carbonsäure-ethylester werden in 40 ml Methanol gelöst und bei 20°C läßt man 7,6 g einer Lösung von 0,0366 Mol Natriummethylat in Methanol zutropfen. Die Mischung wird 12 Stunden bei 55°C gerührt. Dann wird im Wasserstrahlvakuum eingeengt, der Rückstand in 12 ml Wasser aufgenommen und durch Zugabe von konz. Salzsäure unter Eiskühlung der pH-Wert auf 6 eingestellt. Das hierbei kristallin angefallene Produkt wird durch Absaugen isoliert.

Man erhält 3,47 g (69,5% der Theorie) 5-Propoxy-2,4-dihydro-3H-1,2,4-triazol-3-on vom Schmelzpunkt 156°C.

### Beispiel (V-2)

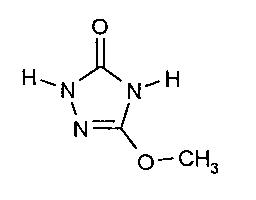

19,5 g (0,115 Mol) N'-(α-Amino-α-methoxy-methylen)-hydrazin-N-carbonsäureethylester (Gehalt: 94,6%) werden in einem Gemisch aus 20 ml Methanol und 30 ml Wasser vorgelegt und nach Zugabe von 10,8 g 45%iger wässriger Natronlauge (0,12 Mol NaOH) wird die Mischung 16 Stunden bei 55°C gerührt. Dann wird im Wasserstrahlvakuum eingeengt, der Rückstand in 30 ml Wasser aufgenommen, unter Eiskühlung mit konz. Salzsäure angesäuert und das kristallin angefallene Produkt durch Absaugen isoliert.

Man erhält 10,0 g (76% der Theorie) 5-Methoxy-2,4-dihydro-3H-1,2,4-triazol-3-on.

### Beispiel (V-3): Stufen 1 und 2 im Eintopfverfahren

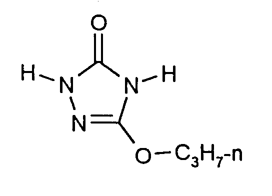

50 g (0,345 Mol) Iminokohlensäure-di-n-propylester (Gehalt: 99,2 %) und 33,7 g (0,314 Mol) Carbazinsäure-ethylester (Gehalt: 97 %) werden in 130 ml n-Propanol vorgelegt, und innerhalb von 1,5 Stunden wird bei Raumtemperatur eine Lösung von 1,6 g (0,0157 Mol) Pivalinsäure in 20 ml n-Propanol zugetropft. Nach vollendeter Zugabe wird über Nacht bei Raumtemperatur nachgerührt und anschließend werden 73,4 g (0,345 Mol NaOCH₃) einer methanolischen Lösung von Natriummethylat hinzugetropft; dann läßt man das Reaktionsgemisch 22 Stunden bei 55-60°C nachrühren. Nachfolgend wird das Lösungsmittel im Vakuum entfernt und der Rückstand wird mit 40 ml Eiswasser und 160 ml n-Buttersäurenitril versetzt; dieses Gemisch wird unter Kühlung durch Zugabe von konzentrierter Salzsäure angesäuert und auf 85°C erhitzt, sodann werden die beiden Phasen getrennt. Die wäßrige Phase wird noch zweimal mit je 40 ml n-Buttersäurenitril bei 85°C ausgerührt (extrahiert), und die vereinigten organischen Phasen werden mit 15 ml gesättigter Kochsalzlösung gewaschen und im Vakuum eingedampft. Den zurückbleibenden Feststoff verrührt man mit 300 ml Petrolether und filtriert das Produkt ab.

Man erhält 45,7 g (91,1 % der Theorie, über beide Stufen, bezogen auf eingesetzten Carbazinsäure-ethylester) 5-Propoxy-2,4-dihydro-3H-1,2,4-triazol-3-on (Gehalt: 89,5 %).

## Patentansprüche

1. Verfahren zur Herstellung von Alkoxytriazolinonen der allgemeinen Formel (I) 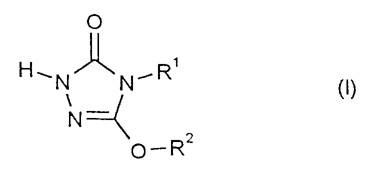 in welcher
R¹ für jeweils gegebenenfalls durch Cyano, Halogen oder C₁-C₄-Alkoxy substituiertes Alkyl, Alkenyl oder Alkinyl mit jeweils bis zu 6 Kohlenstoffatomen, für jeweils gegebenenfalls durch Halogen oder C₁-C₄-Alkyl substituiertes Cycloalkyl oder Cycloalkylalkyl mit jeweils 3 bis 6 Kohlenstoffatomen im Cycloalkylteil und gegebenenfalls 1 bis 4 Kohlenstoffatomen im Alkylteil, oder für jeweils gegebenenfalls durch Carboxy, Cyano, Nitro, Halogen, C₁-C₄-Alkyl, C₁-C₄-Halogenalkyl, C₁-C₄-Alkoxy, C₁-C₄-Halogenalkoxy oder C₁-C₄-Alkoxy-carbonyl substituiertes Aryl oder Arylalkyl mit jeweils 6 oder 10 Kohlenstoffatomen im Arylteil und gegebenenfalls 1 bis 4 Kohlenstoffatomen im Alkylteil steht und
R² für jeweils gegebenenfalls durch Halogen oder C₁-C₄-Alkoxy substituiertes Alkyl, Alkenyl oder Alkinyl mit jeweils bis zu 6 Kohlenstoffatomen, für jeweils gegebenenfalls durch Halogen oder C₁-C₄-Alkyl substituiertes Cycloalkyl oder Cycloalkylalkyl mit jeweils 3 bis 6 Kohlenstoffatomen im Cycloalkylteil und gegebenenfalls 1 bis 4 Kohlenstoffatomen im Alkylteil, oder für jeweils gegebenenfalls durch Carboxy, Cyano, Nitro, Halogen, C₁-C₄-Alkyl, C₁-C₄-Halogenalkyl, C₁-C₄-Alkoxy, C₁-C₄-Halogenalkoxy oder C₁-C₄-Alkoxy-carbonyl substituiertes Aryl oder Arylalkyl mit jeweils 6 oder 10 Kohlenstoffatomen im Arylteil und gegebenenfalls 1 bis 4 Kohlenstoffatomen im Alkylteil steht,
dadurch gekennzeichnet, daß man Iminokohlensäurediester der allgemeinen Formel (II) 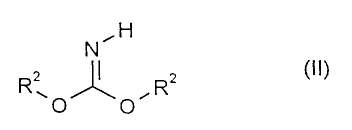 in welcher
R² die oben angegebene Bedeutung hat,
mit Carbazinsäureestern der allgemeinen Formel (III) 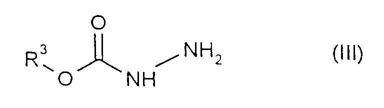 in welcher
R³ für gegebenenfalls durch C₁-C₄-Alkoxy substituiertes C₁-C₄-Alkyl, für Phenyl oder Benzyl steht,
gegebenenfalls in Gegenwart eines Reaktionshilfsmittels und gegebenenfalls in Gegenwart eines Verdünnungsmittels bei Temperaturen zwischen -20°C und +120°C umsetzt ("erste Umsetzungsstufe") und die hierbei gebildeten Semicarbazid-Derivate der allgemeinen Formel (IV) 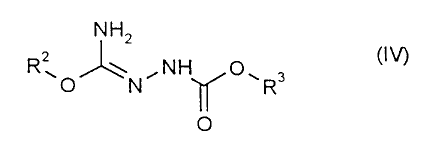 in welcher
R² und R³ die oben angegebene Bedeutung haben,
- und/oder die entsprechenden hierzu tautomeren Verbindungen -,
gegebenenfalls nach Zwischenisolierung, gegebenenfalls in Gegenwart einer Base und gegebenenfalls in Gegenwart eines Verdünnungsmittels bei Temperaturen zwischen 20°C und 150°C cyclisierend kondensiert ("zweite Umsetzungsstufe") und schließlich die so erhaltenen Alkoxytriazolinone der allgemeinen Formel (V) 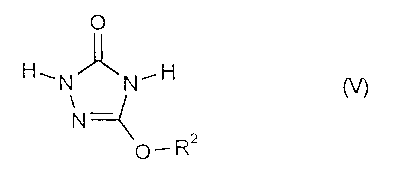 in welcher
R² die oben angegebene Bedeutung hat,
- und/oder die entsprechenden hierzu tautomeren Verbindungen -
mit einem Alkylierungsmittel der allgemeinen Formel (VI)
R¹-X (VI)
worin
X für Halogen oder die Gruppierungen -O-SO₂-O-R¹ oder -O-CO-O-R¹ steht und
R¹ die oben angegebene Bedeutung hat,
gegebenenfalls in Gegenwart einer Base und gegebenenfalls in Gegenwart eines Verdünnungsmittels bei Temperaturen zwischen 0°C und 150°C umsetzt ("dritte Umsetzungsstufe").

2. Verfahren nach Anspruch 1, dadurch gekennzeichnet, daß Verbindungen der Formel (I) hergestellt werden, in welcher
R¹ für jeweils gegebenenfalls durch Cyano, Fluor, Chlor und/oder Brom, Methoxy oder Ethoxy substituiertes Methyl, Ethyl, n- oder i-Propyl, n-, i-, s- oder t-Butyl, für jeweils gegebenenfalls durch Cyano, Fluor, Chlor und/oder Brom substituiertes Propenyl, Butenyl, Propinyl oder Butinyl, für jeweils gegebenenfalls durch Fluor, Chlor, Brom, Methyl oder Ethyl substituiertes Cyclopropyl, Cyclobutyl oder Cyclopropylmethyl, oder für jeweils gegebenenfalls durch Cyano, Fluor, Chlor, Brom, Methyl, Ethyl, Trifluormethyl, Methoxy, Ethoxy, Difluormethoxy, Trifluormethoxy, Methoxycarbonyl oder Ethoxycarbonyl substituiertes Phenyl oder Benzyl steht und
R² für jeweils gegebenenfalls durch Fluor, Chlor und/oder Brom, Methoxy oder Ethoxy substituiertes Methyl, Ethyl, n- oder i-Propyl, n-, i-, s- oder t-Butyl, für jeweils gegebenenfalls durch Cyano, Fluor, Chlor und/oder Brom substituiertes Propenyl, Butenyl, Propinyl oder Butinyl, für jeweils gegebenenfalls durch Fluor, Chlor, Methyl oder Ethyl substituiertes Cyclopropyl oder Cyclopropylmethyl, oder für jeweils gegebenenfalls durch Cyano, Fluor, Chlor, Brom, Methyl, Ethyl, Trifluormethyl, Methoxy, Ethoxy, Difluormethoxy, Trifluormethoxy, Methoxycarbonyl oder Ethoxycarbonyl substituiertes Phenyl oder Benzyl steht.

3. Verfahren nach Anspruch 1, dadurch gekennzeichnet, daß man die erste Umsetzungsstufe bei Temperaturen zwischen -10°C und +90°C, insbesondere zwischen 0°C und +60°C, durchführt.

4. Verfahren nach Anspruch 1, dadurch gekennzeichnet, daß man die zweite Umsetzungsstufe bei Temperaturen zwischen +30°C und +90°C, insbesondere zwischen +40°C und +80°C, durchführt.

5. Verfahren nach Anspruch 1, dadurch gekennzeichnet, daß man die dritte Umsetzungsstufe bei Temperaturen zwischen +30°C und +90°C, insbesondere zwischen +40°C und +80°C, durchführt.

6. Verfahren nach Anspruch 1, dadurch gekennzeichnet, daß als Iminokohlensaurediester der Formel (II) Iminokohlensauredimethylester oder Iminokohlensäuredipropylester eingesetzt wird.

7. Verfahren nach Anspruch 1, dadurch gekennzeichnet, daß man als Carbazinsäureester der Formel (III) Carbazinsäureethylester einsetzt.

8. Semicarbazid-Derivate der Formel (IV) 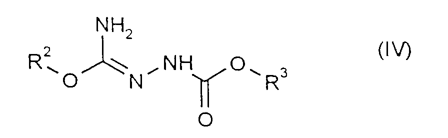 in welcher
R² für jeweils gegebenenfalls durch Halogen oder C₁-C₄-Alkoxy substituiertes Alkyl, Alkenyl oder Alkinyl mit jeweils bis zu 6 Kohlenstoffatomen, für jeweils gegebenenfalls durch Halogen oder C₁-C₄-Alkyl substituiertes Cycloalkyl oder Cycloalkylalkyl mit jeweils 3 bis 6 Kohlenstoffatomen im Cycloalkylteil und gegebenenfalls 1 bis 4 Kohlenstoffatomen im Alkylteil, oder für jeweils gegebenenfalls durch Carboxy, Cyano, Nitro, Halogen, C₁-C₄-Alkyl, C₁-C₄-Halogenalkyl, C₁-C₄-Alkoxy, C₁-C₄-Halogenalkoxy oder C₁-C₄-Alkoxycarbonyl substituiertes Aryl oder Arylalkyl mit jeweils 6 oder 10 Kohlenstoffatomen im Arylteil und gegebenenfalls 1 bis 4 Kohlenstoffatomen im Alkylteil steht und
R³ für gegebenenfalls durch C₁-C₄-Alkoxy substituiertes C₁-C₄-Alkyl, für Phenyl oder Benzyl steht,
ausgenommen diejenigen Verbindungen der Formel (IV), worin
R² für Phenyl und R³ für Methyl oder t-Butyl steht;
R² für 2.2.2-Trichlorethyl und R³ für Methyl, Ethyl oder t-Butyl steht; und
R² für 2.2.2-Trifluorethyl und R³ für Methyl, Ethyl oder t-Butyl steht.

9. Semicarbazid-Derivate gemäß Anspruch 8, nämlich
(a) N'-(α-Amino-α-methoxy-methylen)-hydrazin-N-carbonsäure-ethylester, und
(b) N'-(α-Amino-α-propoxy-methylen)-hydrazin-N-carbonsäure-ethylester.

10. Alkoxytriazolinone der Formel (V) 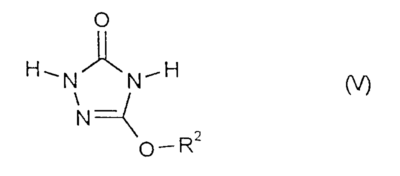 in welcher
R² für jeweils gegebenenfalls durch Halogen oder C₁-C₄-Alkoxy substituiertes Alkyl, Alkenyl oder Alkinyl mit jeweils bis zu 6 Kohlenstoffatomen, für jeweils gegebenenfalls durch Halogen oder C₁-C₄-Alkyl substituiertes Cycloalkyl oder Cycloalkylalkyl mit jeweils 3 bis 6 Kohlenstoffatomen im Cycloalkylteil und gegebenenfalls 1 bis 4 Kohlenstoffatomen im Alkylteil, oder für jeweils gegebenenfalls durch Carboxy, Cyano, Nitro, Halogen, C₁-C₄-Alkyl, C₁-C₄-Halogenalkyl, C₁-C₄-Alkoxy, C₁-C₄-Halogenalkoxy oder C₁-C₄-Alkoxy-carbonyl substituiertes Aryl oder Arylalkyl mit jeweils 6 oder 10 Kohlenstoffatomen im Arylteil und gegebenenfalls 1 bis 4 Kohlenstoffatomen im Alkylteil steht,
ausgenommen diejenigen Verbindungen der Formel (V), worin
R² für Methyl, Ethyl, Phenyl, 4-Methylphenyl, 2,4-Dimethylphenyl oder 4-t-Butylphenyl steht.

11. 5-Propoxy-2,4-dihydro-3H-1,2,4-triazol-3-on gemäß Anspruch 10.

## Claims

1. Process for the preparation of alkoxytriazolinones of the general formula (I) 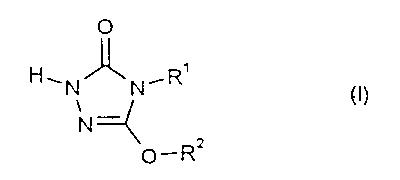 in which
R¹ represents alkyl, alkenyl or alkinyl, each of which has up to 6 carbon atoms and each of which is optionally substituted by cyano, halogen or C₁-C₄-alkoxy, or represents cycloalkyl or cycloalkylalkyl, each of which has 3 to 6 carbon atoms in the cycloalkyl moiety and, if appropriate, 1 to 4 carbon atoms in the alkyl moiety and each of which is optionally substituted by halogen or C₁-C₄-alkyl, or represents aryl or arylalkyl, each of which has 6 or 10 carbon atoms in the aryl moiety and, if appropriate, 1 to 4 carbon atoms in the alkyl moiety and each of which is optionally substituted by carboxyl, cyano, nitro, halogen, C₁-C₄-alkyl, C₁-C₄-halogenoalkyl, C₁-C₄-alkoxy, C₁-C₄-halogenoalkoxy or C₁-C₄-alkoxy-carbonyl, and
R² represents alkyl, alkenyl or alkinyl, each of which has up to 6 carbon atoms and each of which is optionally substituted by halogen or C₁-C₄-alkoxy, or represents cycloalkyl or cycloalkylalkyl, each of which has 3 to 6 carbon atoms in the cycloalkyl moiety and, if appropriate, 1 to 4 carbon atoms in the alkyl moiety and each of which is optionally substituted by halogen or C₁-C₄-alkyl, or represents aryl or arylalkyl, each of which has 6 or 10 carbon atoms in the aryl moiety and, if appropriate, 1 to 4 carbon atoms in the alkyl moiety and each of which is optionally substituted by carboxyl, cyano, nitro, halogen, C₁-C₄-alkyl, C₁-C₄-halogenoalkyl, C₁-C₄-alkoxy, C₁-C₄-halogenoalkoxy or C₁-C₄-alkoxy-carbonyl,
characterized in that
iminocarbonic diesters of the general formula (II) 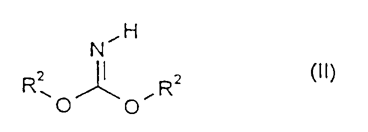 in which
R² has the abovementioned meaning
are reacted with carbazinic esters of the general formula (III) 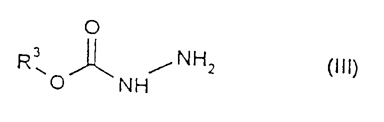 in which
R³ represents C₁-C₄-alkyl which is optionally substituted by C₁-C₄-alkoxy, or represents phenyl or benzyl,
if appropriate in the presence of a reaction auxiliary and if appropriate in the presence of a diluent at temperatures between -20°C and +120°C ("first reaction step") and the semicarbazide derivatives formed in this process of the general formula (IV) 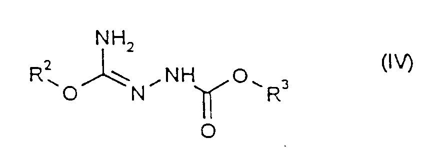 in which
R² and R³ have the abovementioned meaning
- and/or the corresponding tautomeric compounds -
are subjected to a cyclizing condensation reaction, at temperatures between 20°C and 150°C, if appropriate after intermediate isolation, if appropriate in the presence of a base and if appropriate in the presence of a diluent ("second reaction step") and, finally, reacting the resulting alkoxytriazolinones of the general formula (V) 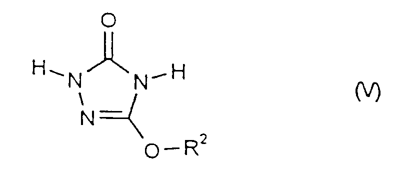 in which
R² has the abovementioned meaning
- and/or the corresponding, tautomeric compounds -
with an alkylating agent of the general formula (VI)
R¹-X (VI)
in which
X represents halogen or the groups -O-SO₂-O-R¹ or -O-CO-O-R¹ and
R¹ has the abovementioned meaning
at temperatures between 0°C and 150°C, if appropriate in the presence of a base and if appropriate in the presence of a diluent ("third reaction step").

2. Process according to Claim 1, characterized in that compounds of the formula (I) are prepared in which
R¹ represents methyl, ethyl, n- or i-propyl or n-, i-, s- or t-butyl, each of which is optionally substituted by cyano, fluorine, chlorine and/or bromine, methoxy or ethoxy, or represents propenyl, butenyl, propinyl or butinyl, each of which is optionally substituted by fluorine, chlorine, bromine, methyl or ethyl, or represents cyclopropyl, cyclobutyl or cyclopropylmethyl, each of which is optionally substituted by fluorine, chlorine, bromine, methyl or ethyl, or represents phenyl or benzyl, each of which is optionally substituted by cyano, fluorine, chlorine, bromine, methyl, ethyl, trifluoromethyl, methoxy, ethoxy, difluoromethoxy, trifluoromethoxy, methoxycarbonyl or ethoxycarbonyl, and
R² represents methyl, ethyl, n- or i-propyl or n-, i-, s- or t-butyl, each of which is optionally substituted by fluorine, chlorine and/or bromine, methoxy or ethoxy, or represents propenyl, butenyl, propinyl or butinyl, each of which is optionally substituted by fluorine, chlorine, bromine, methyl or ethyl, or represents cyclopropyl or cyclopropylmethyl, each of which is optionally substituted by fluorine, chlorine, bromine, methyl or ethyl, or represents phenyl or benzyl, each of which is optionally substituted by cyano, fluorine, chlorine, bromine, methyl, ethyl, trifluoromethyl, methoxy, ethoxy, difluoromethoxy, trifluoromethoxy, methoxycarbonyl or ethoxycarbonyl.

3. Process according to Claim 1, characterized in that the first reaction step is carried out at temperatures between -10°C and +90°C, in particular between 0°C and +60°C.

4. Process according to Claim 1, characterized in that the second reaction step is carried out at temperatures between +30°C and +90°C, in particular between +40°C and +80°C.

5. Process according to Claim 1, characterized in that the third reaction step is carried out at temperatures between +30°C and +90°C, in particular between +40°C and +80°C.

6. Process according to Claim 1, characterized in that the iminocarbonic diester of the formula (II) employed is dimethyl iminocarbonate or dipropyl iminocarbonate.

7. Process according to Claim 1, characterized in that the carbazinic ester of the formula (III) employed is ethyl carbazinate.

8. Semicarbazide derivatives of the formula (IV) 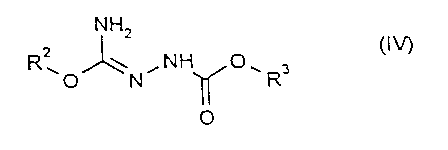 in which
R² represents alkyl, alkenyl or alkinyl, each of which has up to 6 carbon atoms and each of which is optionally substituted by halogen or C₁-C₄-alkoxy, or represents cycloalkyl or cycloalkylalkyl, each of which has 3 to 6 carbon atoms in the cycloalkyl moiety and, if appropriate, 1 to 4 carbon atoms in the alkyl moiety and each of which is optionally substituted by halogen or C₁-C₄-alkyl, or represents aryl or arylalkyl, each of which has 6 or 10 carbon atoms in the aryl moiety and, if appropriate, 1 to 4 carbon atoms in the alkyl moiety and each of which is optionally substituted by carboxyl, cyano, nitro, halogen, C₁-C₄-alkyl, C₁-C₄-halogenoalkyl, C₁-C₄-alkoxy, C₁-C₄-halogenoalkoxy or C₁-C₄-alkoxy-carbonyl, and
R³ represents C₁-C₄-alkyl which is optionally substituted by C₁-C₄-alkoxy, or represents phenyl or benzyl,
with the exception of those compounds of formula (IV) in which
R² represents phenyl and R³ represents methyl or t-butyl;
R² represents 2.2.2-trichloroethyl and R³ represents methyl, ethyl or t-butyl; and
R² represents 2.2.2-trifluoroethyl and R³ represents methyl, ethyl or t-butyl.

9. Semicarbazide derivatives according to Claim 8, namely
(a) ethyl N'-("-amino-"-methoxy-methylene)-hydrazine-N-carboxylate and
(b) ethyl N'-("-amino-"-propoxy-methylene)-hydrazine-N-carboxylate.

10. Alkoxytriazolinones of the formula (V) 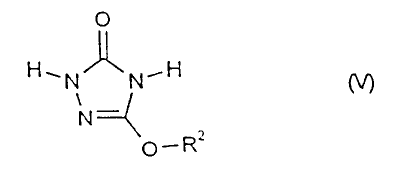 in which
R² represents alkyl, alkenyl or alkinyl, each of which has up to 6 carbon atoms and each of which is optionally substituted by halogen or C₁-C₄-alkoxy, or represents cycloalkyl or cycloalkylalkyl, each of which has 3 to 6 carbon atoms in the cycloalkyl moiety and, if appropriate, 1 to 4 carbon atoms in the alkyl moiety and each of which is optionally substituted by halogen or C₁-C₄-alkyl, or represents aryl or arylalkyl, each of which has 6 or 10 carbon atoms in the aryl moiety and, if appropriate, 1 to 4 carbon atoms in the alkyl moiety and each of which is optionally substituted by carboxyl, cyano, nitro, halogen, C₁-C₄-alkyl, C₁-C₄-halogenoalkyl, C₁-C₄-alkoxy, C₁-C₄-halogenoalkoxy or C₁-C₄-alkoxy-carbonyl,
with the exception of those compounds of the formula (V) in which
R² represents methyl, ethyl, phenyl, 4-methylphenyl, 2,4-dimethylphenyl or 4-t-butylphenyl.

11. 5-Propoxy-2,4-dihydro-3H-1,2,4-triazol-3-one according to Claim 10.

## Revendications

1. Procédé de production d'alkoxytriazolinones de formule générale (I) 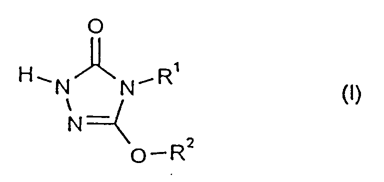 dans laquelle
R¹ représente un groupe alkyle, alcényle ou alcynyle ayant chacun jusqu'à 6 atomes de carbone et portant chacun éventuellement un substituant cyano, halogéno ou alkoxy en C₁ à C₄, un groupe cycloalkyle ou cycloalkylalkyle ayant chacun 3 à 6 atomes de carbone dans la partie cycloalkyle et, le cas échéant, 1 à 4 atomes de carbone dans la partie alkyle et portant chacun, le cas échéant, un substituant halogéno ou alkyle en C₁ à C₄, ou un groupe aryle ou arylalkyle ayant chacun 6 ou 10 atomes de carbone dans la partie aryle et, le cas échéant, 1 à 4 atomes de carbone dans la partie alkyle et portant chacun, le cas échéant, un substituant carboxy, cyano, nitro, halogéno, alkyle en C₁ à C₄, halogénalkyle en C₁ à C₄, alkoxy en C₁ à C₄, halogénalkoxy en C₁ à C₄ ou (alkoxy en C₁ à C₄)-carbonyle, et
R² représente un groupe alkyle, alcényle ou alcynyle ayant chacun jusqu'à 6 atomes de carbone et portant chacun, le cas échéant, un substituant halogéno ou alkoxy en C₁ à C₄, un groupe cycloalkyle ou cycloalkylalkyle ayant chacun 3 à 6 atomes de carbone dans la partie cycloalkyle et, le cas échéant, 1 à 4 atomes de carbone dans la partie alkyle et portant chacun, le cas échéant, un substituant halogéno ou alkyle en C₁ à C₄, ou un groupe aryle ou arylalkyle ayant chacun 6 ou 10 atomes de carbone dans la partie aryle et, le cas échéant, 1 à 4 atomes de carbone dans la partie alkyle et portant chacun, le cas échéant, un substituant carboxy, cyano, nitro, halogéno, alkyle en C₁ à C₄, halogénalkyle en C₁ à C₄, alkoxy en C₁ à C₄, halogénalkoxy en C₁ à C₄ ou (alkoxy en C₁ à C₄)-carbonyle,
caractérisé en ce qu'on fait réagir des diesters d'acide iminocarbonique de formule générale (II) 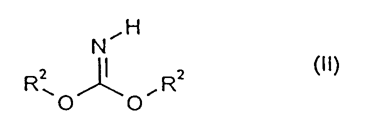 dans laquelle
R² a la définition indiquée ci-dessus,
avec des esters d'acide carbazinique de formule générale (III) 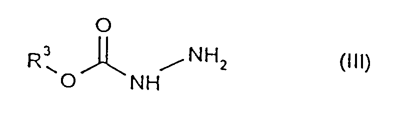 dans laquelle
R³ est un groupe alkyle en C₁ à C₄ éventuellement substitué par un radical alkoxy en C₁ à C₄, un groupe phényle ou benzyle,
le cas échéant en présence d'un auxiliaire de réaction et en la présence éventuelle d'un diluant à des températures comprises entre -20°C et +120°C ("première étape de réaction") et on condense par cyclisation ("deuxième étape de réaction") les dérivés semicarbazides ainsi formés, de formule générale (IV) 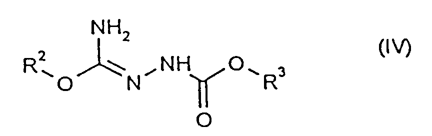 dans laquelle
R² et R³ ont la définition indiquée ci-dessus,
- et/ou les composés correspondants qui en sont tautomères -,
le cas échéant après isolement intermédiaire, éventuellement en présence d'une base et en la présence éventuelle d'un diluant, à des températures comprises entre 20°C et 150°C et, finalement, on fait réagir ("troisième étape de réaction") les alkoxytriazolinones ainsi obtenues de formule générale (V) 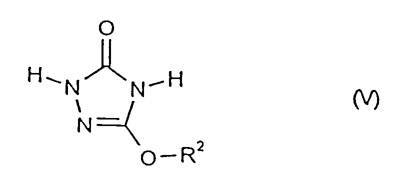 dans laquelle
R² a la définition indiquée ci-dessus,
- et/ou les composés correspondants qui en sont les tautomères -
avec un agent d'alkylation de formule générale (VI)
R¹-X (VI)
dans laquelle
X représente un halogène ou les groupements -O-SO₂-O-R¹ ou -O-CO-O-R¹ et
R¹ a la définition indiquée ci-dessus,
le cas échéant en présence d'une base et en la présence éventuelle d'un diluant, à des températures comprises entre 0°C et 150°C.

2. Procédé suivant la revendication 1, caractérisé en ce qu'on prépare des composés de formule (I), dans laquelle
R¹ représente un groupe méthyle, éthyle, n-propyle, isopropyle, n-butyle, isobutyle, sec. -butyle ou tertio-butyle portant chacun, le cas échéant, un substituant cyano, fluoro, chloro et/ou bromo, méthoxy ou éthoxy, un groupe propényle, butényle, propinyle ou butynyle portant chacun, le cas échéant, un substituant cyano, fluoro, chloro et/ou bromo, un groupe cyclopropyle, cyclobutyle ou cyclopropylméthyle portant chacun, le cas échéant, un substituant fluoro, chloro, bromo, méthyle ou éthyle, ou un groupe phényle ou benzyle portant chacun, le cas échéant, un substituant cyano, fluoro, chloro, bromo, méthyle, éthyle, trifluorométhyle, méthoxy, éthoxy, difluorométhoxy, trifluorométhoxy, méthoxycarbonyle ou éthoxycarbonyle et
R² représente un groupe méthyle, éthyle, n-propyle, isopropyle, n-butyle, isobutyle, sec.-butyle ou tertio-butyle portant chacun, le cas échéant, un substituant fluoro, chloro et/ou bromo, méthoxy ou éthoxy, un groupe propényle, butényle, propinyle ou butynyle portant chacun, le cas échéant, un substituant cyano, fluoro, chloro et/ou bromo, un groupe cyclopropyle ou cyclopropylméthyle portant chacun, le cas échéant, un substituant fluoro, chloro, méthyle ou éthyle, ou un groupe phényle ou benzyle portant chacun, le cas échéant, un substituant cyano, fluoro, chloro, bromo, méthyle, éthyle, trifluorométhyle, méthoxy, éthoxy, difluorométhoxy, trifluorométhoxy, méthoxycarbonyle ou éthoxycarbonyle.

3. Procédé suivant la revendication 1, caractérisé en ce qu'on conduit la première étape de réaction à des températures comprises entre -10°C et +90°C, notamment entre 0°C et +60°C.

4. Procédé suivant la revendication 1, caractérisé en ce qu'on conduit la deuxième étape de réaction à des températures comprises entre +30°C et +90°C, notamment entre +40°C et +80°C.

5. Procédé suivant la revendication 1, caractérisé en ce qu'on conduit la troisième étape de réaction à des températures comprises entre +30°C et +90°C, notamment entre +40°C et +80°C.

6. Procédé suivant la revendication 1, caractérisé en ce qu'on utilise comme diester d'acide iminocarbonique de formule (II) l'ester de diméthyle d'acide iminocarbonique ou l'ester de dipropyle d'acide iminocarbonique.

7. Procédé suivant la revendication 1, caractérisé en ce qu'on utilise comme ester d'acide carbazinique de formule (III) l'ester éthylique d'acide carbazinique.

8. Semicarbazides de formule (IV) 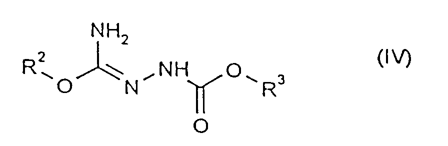 dans laquelle
R² est un groupe alkyle, alcényle ou alcynyle ayant chacun jusqu'à 6 atomes de carbone et portant chacun, le cas échéant, un substituant halogéno ou alkoxy en C₁ à C₄, un groupe cycloalkyle ou cycloalkylalkyle ayant chacun 3 à 6 atomes de carbone dans la partie cycloalkyle et, le cas échéant, 1 à 4 atomes de carbone dans la partie alkyle et portant chacun, le cas échéant, un substituant halogéno ou alkyle en C₁ à C₄, ou un groupe aryle ou arylalkyle ayant chacun 6 ou 10 atomes de carbone dans la partie aryle et, le cas échéant, 1 à 4 atomes de carbone dans la partie alkyle et portant chacun, le cas échéant, un substituant carboxy, cyano, nitro, halogéno, alkyle en C₁ à C₄, halogénalkyle en C₁ à C₄, alkoxy en C₁ à C₄, halogénalkoxy en C₁ à C₄ ou (alkoxy en C₁ à C₄)-carbonyle, et
R³ représente un groupe alkyle en C₁ à C₄ éventuellement substitué par un radical alkoxy en C₁ à C₄,
un groupe phényle ou benzyle,
excepté les composés de formule (IV), dans laquelle
R² est un groupe phényle et R³ est un groupe méthyle ou tertio-butyle ;
R² est un groupe 2.2.2-trichloréthyle et R³ est un groupe méthyle, éthyle ou tertio-butyle ; et
R² est un groupe 2.2.2-trifluoréthyle et R³ est un groupe méthyle, éthyle ou tertio-butyle.

9. Semicarbazides suivant la revendication 8, à savoir
(a) l'ester éthylique d'acide N'-(α-amino-α-méthoxyméthylène)-hydrazine-N-carboxylique, et
(b) l'ester éthylique d'acide N'(α-amino-α-propoxyméthylène)-hydrazine-N-carboxylique.

10. Alkoxytriazolinones de formule (V) 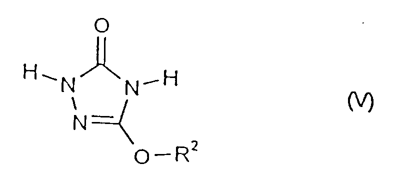 dans laquelle
R² représente un groupe alkyle, alcényle ou alcynyle ayant chacun jusqu'à 6 atomes de carbone et portant chacun, le cas échéant, un substituant halogéno ou alkoxy en C₁ à C₄, un groupe cycloalkyle ou cycloalkylalkyle ayant chacun 3 à 6 atomes de carbone dans la partie cycloalkyle et, le cas échéant, 1 à 4 atomes de carbone dans la partie alkyle et portant chacun, le cas échéant, un substituant halogéno ou alkyle en C₁ à C₄, ou un groupe aryle ou arylalkyle ayant chacun 6 ou 10 atomes de carbone dans la partie aryle et, le cas échéant, 1 à 4 atomes de carbone dans la partie alkyle et portant chacun, le cas échéant, un substituant carboxy, cyano, nitro, halogéno, alkyle en C₁ à C₄, halogénalkyle en C₁ à C₄, alkoxy en C₁ à C₄, halogénalkoxy en C₁ à C₄ ou (alkoxy en C₁ à C₄)-carbonyle,
excepté les composés de formule (V), dans laquelle
R² est un groupe méthyle, éthyle, phényle, 4-méthylphényle, 2,4-diméthylphényle ou 4-tertio-butylphényle.

11. La 5-propoxy-2,4-dihydro-3H-1,2,4-triazole-3-one suivant la revendication 10.
